# EUROPÄISCHE PATENTSCHRIFT

(11) **EP 3 433 502 B1**
(45) Veröffentlichungstag und Bekanntmachung des Hinweises auf die Patenterteilung: **05.05.2021**
(21) Anmeldenummer: 17712992.1
(22) Anmeldetag: 23.03.2017
(51) Int. Cl.: F16C 1/28, F16C 3/035, F16C 29/04, A61B 17/00, B26D 1/04, F16C 33/40, B27B 19/00, A61B 17/14

(54) **MEDIZINISCHE VORRICHTUNG**
MEDICAL DEVICE
DISPOSITIF MÉDICAL

(30) Priorität: 23.03.2016 DE 102016105431
(43) Veröffentlichungstag der Anmeldung: 30.01.2019
(73) Patentinhaber: Aesculap AG, 78532 Tuttlingen (DE)
(72) Erfinder: MACHILL, Martin, 78604 Rietheim-Weilheim (DE)
(74) Vertreter: Hoeger, Stellrecht & Partner Patentanwälte mbB
(86) Internationale Anmeldenummer: PCT/EP2017/056903
(87) Internationale Veröffentlichungsnummer: WO 2017/162786

(56) Entgegenhaltungen:
- EP-A1- 2 208 901
- DE-A1- 2 227 312
- DE-A1- 2 849 760
- DE-A1-102014 225 134
- DE-U- 6 901 870
- JP-A- 2014 001 748
- JP-U- S5 215 645
- JP-Y1- S 468 483

## Beschreibung

Die vorliegende Erfindung betrifft eine medizinische Vorrichtung mit einem linear beweglich gelagerten Maschinenelement und mindestens einer mindestens ein Kugellager umfassenden, eine Längsachse definierenden Linearführung zum Führen einer linearen oszillierenden Hin- und Herbewegung des Maschinenelements parallel zur Längsachse mit einer hohen Oszillationsfrequenz, welches mindestens eine Kugellager eine Mehrzahl von Kugeln umfasst, die in korrespondierenden Kugelaufnahmen beweglich gehalten sind und die das Maschinenelement führen, wobei die Mehrzahl von Kugelaufnahmen in Form von Langlöchern ausgebildet ist, wobei die Mehrzahl von Langlöchern eine freie Bewegungslänge für die jeweils in ihnen beweglich angeordnete Kugel aufweist und wobei die freie Bewegungslänge mindestens einem halben Hub des Maschinenelements relativ zur Linearführung entspricht, wobei die Langlöcher jeweils eine Langlochlängsachse definieren.

Üblicherweise sind die am Markt verfügbaren Linearführungen für medizinische Vorrichtungen der eingangs beschriebenen Art derart ausgebildet, dass die Kugeln in korrespondierenden Kugelaufnahmen in Form von Bohrungen eines Lager- oder Kugelkäfigs, nachstehend auch nur als Lagerkäfig bezeichnet, eingelassen sind. Ein Problem solcher medizinischer Vorrichtungen ist es insbesondere, das Maschinenelement mit einer hohen Oszillationsfrequenz hin und her zu bewegen. Dabei wird der Lagerkäfig mit einer oszillierenden Hin- und Herbewegung, die auch als Hubbewegung bezeichnet wird, mitbewegt. Aufgrund der Massenträgheit kann es im Umkehrpunkt der Oszillationsbewegung zu einem Rutschen oder Schieben der Kugeln auf ihren jeweiligen Laufpartnern kommen, beispielsweise dem Maschinenelement selbst. Dadurch können die Kugeln in unerwünschter Weise verschleißen, wodurch eine Qualität der Linearführung signifikant leidet und sich eine Standzeit der Vorrichtung verringert.

Aus der DE 10 2014 225 134 A1 ist ein Wälzlager bekannt. In der EP 2 208 901 A1 sind ein Wälzkörperkäfig für eine Möbelauszugsführung und ein Verfahren zur Montage eines Wälzkörperkäfigs beschrieben. Linearkugellager sind in der JP 2014-001748 A offenbart. Kugellager sind aus der JP S46 8483 Y bekannt. In der JP S52 15645 U sind Kugellager mit schräg zur Längsachse verlaufenden Aufnahmen beschrieben. Ein Trenngerät ist in der DE 28 49 760 A1 offenbart. Aus der DE 2 227 312 A1 ist ein Längslager bekannt, das die strukturellen Merkmale der Präambel, jedoch nicht ausdrücklich eine medizinische Vorrichtung zeigt. Kugellager zur Aufnahme von kleinen Axialbewegungen sind in der DE 69 01 870 U offenbart. Es ist daher eine Aufgabe der vorliegenden Erfindung, bei einer medizinischen Vorrichtung der eingangs beschriebenen Art die Führung des Maschinenelements zu verbessern.

Diese Aufgabe wird bei einer medizinischen Vorrichtung der eingangs beschrieben Art erfindungsgemäß dadurch gelöst, dass die Langlochlängsachse quer zur Längsachse verläuft, dass die Langlochlängsachsen mit der Längsachse einen Winkel in einem Bereich zwischen 0° und 90° einschließen und dass die medizinische, insbesondere sterilisierbare, Vorrichtung ausgebildet ist, um mit einer Reinigungsflüssigkeit gereinigt zu werden.

Die erfindungsgemäß vorgeschlagene Lösung ermöglicht es insbesondere, eine Bewegung der Kugeln in den Kugelaufnahmen von einer Bewegung der Kugelaufnahmen zu entkoppeln. Anders als bei herkömmlichen Linearführungen, bei denen die Kugeln in Bohrungen eines Lagerkäfigs angeordnet sind, können sich die Kugeln in den langlochförmigen Kugelaufnahmen hin und her bewegen und so an einem oder beiden Gleitpartnern abrollen, beispielsweise dem geführten Maschinenelement. Bei entsprechender Dimensionierung der Kugelaufnahmen, insbesondere einer entsprechenden Länge der Langlöcher, kann so ein die Kugelaufnahmen umfassender Lagerkäfig im Wesentlichen unbewegt verbleiben, und zwar trotz einer Bewegung des Maschinenelements parallel zur Längsachse. Auf diese Weise lässt sich ein Verschleiß der Linearführung minimieren und somit eine Standzeit derselben und der medizinischen Vorrichtung erhöhen. Die Langlöcher ermöglichen es also insbesondere, die zu bewegenden Massen zu reduzieren. Dies könnte zwar auch dadurch erreicht werden, dass der Lagerkäfig nicht aus einem Stahl ausgebildet wird, sondern aus einem leichteren Werkstoff, beispielsweise einem Kunststoff oder aus einer Keramik. Allerdings ist insbesondere bei Kunststoffen ein Verschleiß relativ groß und Keramiken sind zu diesem Zweck nur aufwändig herstellbar und teuer. Die Erfindung ermöglicht es also insbesondere, herkömmliche Lagerkäfige aus Stahl zu verwenden, ohne diese bewegen zu müssen. Eine Masse des Lagerkäfigs spielt also für eine oszillierende Bewegung des Maschinenelements und dessen lineare Führung praktisch keine Rolle. Unter einem Langloch ist hier insbesondere eine Durchbrechung zu verstehen, die in einer ersten Richtung eine größere Ausdehnung aufweist als in einer zweiten Richtung quer zur ersten Richtung, sodass die im Langloch gelagerte Kugel im Wesentlichen nur parallel zur ersten Richtung bewegt werden , also nur hin- und her- rollen, kann. Ferner ermöglicht es die Anordnung der Kugeln in Kugelaufnahmen, die in Form von Langlöchern ausgebildet sind, die medizinische Vorrichtung einfacher zu reinigen. Reinigungsflüssigkeit kann deutlich besser zwischen die Kugeln und die Kugelaufnahmen eines Lagerkäfigs gelangen. Gemäß der Erfindung ist vorgesehen, dass die Mehrzahl von Langlöchern eine freie Bewegungslänge für die jeweils in ihnen beweglich angeordnete Kugel aufweist und dass die freie Bewegungslänge mindestens einem halben Hub des Maschinenelements relativ zur Linearführung entspricht. Ein Hub, auch als Hublänge bezeichnet, des Maschinenelements entspricht einer Strecke, längs derer das Maschinenelement aufgrund einer Bewegung parallel zur Längsachse hin und her oszilliert. Die vorgeschlagene freie Bewegungslänge von mindestens einem halben Hub des Maschinenelements stellt sicher, dass die Kugeln mit aufeinander zu weisenden inneren Endflächen der Langlöcher der Kugelaufnahmen nicht zwingend in Kontakt treten müssen, wenn sie an ihren beiden Lagerpartnern, also insbesondere dem Maschinenelement, abrollen. So lässt sich eine Länge der Langlöcher minimieren und trotzdem der gewünschte Effekt erreichen, nämlich insbesondere eine Bewegung des Lagerkäfigs selbst zu vermeiden und eine Reinigbarkeit des Kugellagers zu verbessern. Vorteilhaft ist es, dass die Langlöcher jeweils eine Langlochlängsachse definieren und dass die Langlochlängsachse quer zur Längsachse verläuft. Wenn insbesondere keine Rotation des Lagerkäfigs um die Längsachse gewünscht wird, ist es vorteilhaft, wenn die Langlöcher mit ihrer Langlochlängsachse parallel zur Längsachse ausgerichtet sind. Dagegen lässt sich ein sogenanntes Einlaufen von Kugeln am Maschinenelement, also das Ausbilden von Schleifspuren durch eine Gleitbewegung statt der gewünschten Abrollbewegung der Kugeln relativ zum Maschinenelement, verhindern, indem die Langlochlängsachsen der Kugelaufnahmen quer zur Längsachse verlaufen. Quer bedeutet hier insbesondere nicht senkrecht, also unter einem Winkel zwischen 0° und 90°, welcher jedoch vorzugsweise deutlich kleiner als 90° ist, insbesondere kleiner als 50°.

Günstig ist es, wenn das Maschinenelement mindestens einen rotationssymmetrischen Maschinenelementabschnitt umfasst, welcher in der mindestens einen Linearführung geführt ist. Der rotationssymmetrische Maschinenelementabschnitt ermöglicht insbesondere auch eine Verdrehung relativ zur Linearführung, falls dies erforderlich sein sollte. Zudem ist eine Ausbildung eines diesen Maschinenelementabschnitt umgebenden Kugellagers auf einfache Weise zu realisieren.

Besonders einfach herzustellen wird der rotationssymmetrische Maschinenelementabschnitt, wenn er einen kreisförmigen Querschnitt aufweist.

Für besondere Arten medizinischer Vorrichtungen kann es günstig sein, wenn das Maschinenelement in Form eines Stößels oder eines Kolbens ausgebildet ist. Beispielsweise lassen sich so Oszillationssägen in Form von Stichsägen auf einfache Weise ausbilden.

Der Aufbau der medizinischen Vorrichtung lässt sich weiter vereinfachen, wenn das mindestens eine Kugellager einen Lagerkäfig umfasst und wenn die Mehrzahl von Kugelaufnahmen im Lagerkäfig ausgebildet ist. Durch die Gestaltung des Lagerkäfigs lassen sich dann die Kugeln in gewünschter Weise anordnen, sodass sie das oszillierend bewegte Maschinenelement in gewünschter Weise führen können.

Um eine definierte Rotation des Lagerkäfigs um die Längsachse zu erzwingen, ist es vorteilhaft, wenn die Langlochlängsachsen mit der Längsachse einen Drallwinkel in einem Bereich von 0° bis etwa 30° einschließen. Eine solche Ausgestaltung ermöglicht es insbesondere, bei einer oszillierenden Bewegung des Maschinenelements den Lagerkäfig durch eine Bewegung der Kugeln in den langlochartigen Kugelaufnahmen auch um die Längsachse zu verdrehen.

Um einen Verschleiß der Linearführung weiter zu minimieren, ist es günstig, wenn die Langlöcher eine freie Bewegungslänge aufweisen, die mindestens 10 % größer ist als der halbe Hub. Insbesondere kann die freie Bewegungslänge mindestens 30 % größer sein als der halbe Hub. So kann ein Kontakt der Kugeln mit inneren Endflächen der Langlöcher der Kugelaufnahmen im Wesentlichen sicher vermieden werden.

Vorzugsweise ist der Lagerkäfig rotierbar um die Längsachse an der Linearführung angeordnet. So kann erreicht werden, dass sich keine Laufspuren der Kugeln am Maschinenelement durch eine oszillierende Hin- und Herbewegung desselben ausbilden können.

Ferner kann es vorteilhaft sein, wenn das mindestens eine Kugellager eine den Lagerkäfig umgebende Lagerhülse aufweist zum Begrenzen einer Bewegung der Kugeln in radialer Richtung von der Längsachse weg. Die Lagerhülse kann insbesondere einen Lagerpartner für die Kugeln bilden, an dem diese abrollen können. Zudem lässt sich so das Kugellager auf einfache Weise herstellen und in gewünschte Ausnehmungen an medizinischen Vorrichtungen einsetzen, beispielsweise in für die Lagerhülse vorgesehene Rücksprünge. Die Kugeln können also beispielsweise am Maschinenelement einerseits und an der Lagerhülse andererseits abrollen, sodass sich das Maschinenelement und die Lagerhülse relativ zueinander um eine Hublänge bewegen können, die Kugeln sich jedoch lediglich um die freie Bewegungslänge relativ zum Maschinenelement und zur Lagerhülse bewegen, welche Bewegungslänge der halben Hublänge und damit dem halben Hub des Maschinenelements relativ zur Linearführung entspricht.

Vorteilhaft ist es, wenn die Mehrzahl von Langlöchern eine Langlochbreite quer zu ihrer jeweiligen Langlochlängsachse aufweist und wenn die Langlochbreite in radialer Richtung auf die Längsachse hin abnimmt. Mit anderen Worten verjüngen sich die Langlöcher in ihrer Breite in Richtung auf die Längsachse hin. Dadurch können sie so ausgebildet werden, dass die Kugeln nicht aus den Langlöchern in Richtung auf die Längsachse hin herausfallen können. Wird der Lagerkäfig beispielsweise von der Lagerhülse umgeben, können so die Kugeln auf einfache Weise gegen ein Herausfallen aus dem Lagerkäfig gesichert werden, und zwar nach innen in Richtung auf die Längsachse hin durch die Verringerung der Langlochbreite und nach außen in Richtung von der Längsachse weg durch die Lagerhülse.

Die Kugeln lassen sich in den Kugelaufnahmen auf einfache Weise gegen Herausfallen in Richtung auf die Längsachse sichern, wenn aufeinander zu weisende seitliche Begrenzungsflächen der Mehrzahl von Langlöchern um einen Konuswinkel gegeneinander geneigt sind.

Günstig ist es, wenn der Konuswinkel von der Längsachse weg weisend geöffnet ist. So lassen sich die Kugeln in den Langlöchern auf einfache Weise gegen ein Herausfallen in Richtung auf die Längsachse sichern.

Um eine optimale Führung des Maschinenelements an der medizinischen Vorrichtung zu ermöglichen, ist es günstig, wenn eine Wandstärke des Lagerkäfigs kleiner als ein Durchmesser der Mehrzahl von Kugeln ist. Insbesondere kann die Wandstärke maximal einem halben Durchmesser der Mehrzahl von Kugeln entsprechen. Auf diese Weise wird sichergestellt, dass die Kugeln sowohl in Richtung auf die Längsachse als auch von der Längsachse weg aus dem Lagerkäfig vorstehen können, um beispielsweise einerseits am Maschinenelement und andererseits an der Lagerhülse abrollen zu können.

Damit eine möglichst gute Führung des Maschinenelements erreicht werden kann, ist es vorteilhaft, wenn der Lagerkäfig mindestens zwei in Umfangsrichtung bezogen auf dessen Längsachse axial voneinander beabstandete, jeweils mehrere Langlöcher umfassende Lochreihen aufweist. Die Lochreihen bilden somit die Längsachse umgebende Ringe. Werden zwei voneinander getrennte Lagerkäfige vorgesehen, kann auch jeder Lagerkäfig nur einen solchen Lochring beziehungsweise eine solche Lochreihe aufweisen.

Vorteilhaft ist es, wenn die Langlöcher benachbarter Lochreihen zueinander fluchtend oder versetzt angeordnet sind. Insbesondere können benachbarte Lochreihen versetzt zueinander angeordnet sein, insbesondere derart, dass Langlöcher einer zweiten Lochreihe um einen Umfangswinkel versetzt zu den Langlöchern einer benachbarten ersten Lochreihe angeordnet sind, welcher Umfangswinkel einem halben Winkelabstand bezogen auf die Längsachse zwischen benachbarten Langlöchern der ersten und/oder zweiten Lochreihe entspricht. So lassen sich besonders kompakte verschleißarme Kugellager ausbilden.

Gemäß einer weiteren bevorzugten Ausführungsform der Erfindung kann vorgesehen sein, dass die Linearführung zwei oder mehr voneinander getrennte Kugellager mit jeweils mindestens einem Lagerkäfig umfasst und dass jeder Lagerkäfig mindestens eine Lochreihe aufweist mit mehreren in Umfangsrichtung bezogen auf dessen Längsachse angeordneten Langlöchern. Diese Ausgestaltung der Linearführung ermöglicht es insbesondere, das Maschinenelement materialsparend zu lagern, insbesondere wenn dies eine bestimmte Länge überschreitet. Es können dann beispielsweise nur in den Endbereichen des Maschinenelements Kugellager mit einer oder mehreren Lochreihen vorgesehen werden. Je weiter voneinander entfernt die Kugellager angeordnet sind, umso stabiler wird eine axiale Führung des Maschinenelements.

Vorteilhaft ist es, wenn der Lagerkäfig eine Radialsicherungseinrichtung aufweist zum Verhindern des Austretens der Kugeln aus den jeweiligen Kugelaufnahmen in einer Richtung von der Längsachse weg oder in einer Richtung auf die Längsachse hin. Die Radialsicherungseinrichtung ermöglicht es insbesondere, das Kugellager als Ganzes handzuhaben, beispielsweise für eine Montage desselben an der medizinischen Vorrichtung, ohne dass die Kugeln des Kugellagers aus den Kugelaufnahmen austreten können. Die Radialsicherungseinrichtung kann insbesondere derart ausgebildet sein, dass die Kugeln zwar nicht aus den Kugelaufnahmen herausfallen können, jedoch aus den Kugelaufnahmen noch so weit vorstehen, dass sie beispielsweise mit dem Maschinenelement und/oder der Lagerhülse in Kontakt treten und an diesen abrollen können, ohne dass der Lagerkäfig das Maschinenelement oder die Lagerhülse berührt.

Auf einfache Weise ausbilden lässt sich die Radialsicherungseinrichtung, wenn diese verstemmte Ränder der Kugelaufnahmen umfasst. Unter einem Verstemmen der Ränder ist insbesondere zu verstehen, dass diese derart verformt sind, dass eine minimale Breite der Kugelaufnahmen kleiner ist als ein Durchmesser der Kugeln, sodass diese nicht herausfallen können. Insbesondere kann das Verstemmen der Ränder der Kugelaufnahmen erfolgen, nachdem die Kugeln zunächst in die Kugelaufnahmen mit unverstemmten Rändern eingesetzt sind. Das Verstemmen der Ränder erfolgt dann beispielsweise bei einer Montage des Kugellagers.

Auf einfache Weise lassen sich die Kugeln in den Kugelaufnahmen sichern, wenn die Radialsicherungseinrichtung eine Deckelhülse umfasst, deren Innendurchmesser zu einem Außendurchmesser des Lagerkäfigs korrespondiert, und wenn die Deckelhülse eine Mehrzahl von langlochartigen Deckeldurchbrechungen aufweist, deren Breite kleiner ist als ein Kugeldurchmesser. Eine solche Deckelhülse erfüllt im Wesentlichen denselben Zweck wie verstemmte Ränder der Kugelaufnahmen. Die Deckeldurchbrechungen der Deckelhülse sind mit ihren Abmessungen so gestaltet, dass die Kugel, die sich in der Kugelaufnahme befindet, nicht durch die Deckeldurchbrechungen hindurchtreten kann. Ferner kann optional auch vorgesehen sein, die Lagerhülse und/oder die Deckelhülse axial am Lagerkäfig zu sichern. Dazu kann beispielsweise die Lagerhülse nach der Montage der Kugeln im Lagerkäfig in Hubrichtung, also parallel zur Längsachse, derart gesichert werden, dass mindestens ein erstes Ende der Lagerhülse rundum oder zumindest abschnittsweise nach innen, also in Richtung auf die Längsachse hin, umverformt wird, beispielsweise durch Bördeln. Ein zweites Ende der Lagerhülse könnte ebenso umverformt werden oder es könnte alternativ auch schon einen festen Bund als Anschlag aufweisen. Die Deckelhülse kann in analoger Weise am Lagerkäfig axial gesichert werden. Durch die Umverformung an mindestens einem der beiden Enden der Lagerhülse und/oder der Deckelhülse wird ein Durchmesser der Lagerhülse und/oder der Deckelhülse verringert, sodass eine Entnahme des mit den Kugeln bestückten Lagerkäfigs parallel zur Längsachse verhindert wird. Die so gebildete Einheit, nämlich der Kugelkäfig mit den Kugeln und der Lagerhülse und/oder der Deckelhülse, kann als Baugruppe einfach gehandhabt und bei Bedarf bei einer Wartung der medizinischen Vorrichtung getauscht werden. Alternativ kann auch eine Deckelung der Lagerhülse und/oder der Deckelhülse vorgesehen werden, und zwar mit einem oder zwei Deckeln in Form von Zusatzteilen, welche in Richtung der Längsachse weisende Öffnungen der Lagerhülse und/oder der Deckelhülse verschließen und so das Herausnehmen des Kugelkäfigs verhindern.

Auf einfache Weise lässt sich die Linearführung ausbilden, wenn die Lagerhülse die Deckelhülse umgebend angeordnet ist. Beispielsweise lassen sich so die Kugeln in die Lageraufnahmen einsetzen und durch Aufschieben der Deckelhülse auf die Lagerhülse in den Lageraufnahmen halten.

Auf einfache und kostengünstige Weise lassen sich Linearführungen ausbilden, wenn das mindestens eine Kugellager aus einem Metall ausgebildet ist. Insbesondere kann der Lagerkäfig aus einem Metall ausgebildet sein. Das Metall kann insbesondere ein Instrumentenstahl sein, der vorzugsweise nicht rostend ausgebildet ist.

Um die medizinische Vorrichtung als medizinische Werkzeugmaschine nutzen zu können, ist es günstig, wenn das Maschinenelement eine Kupplungseinrichtung zum lösbaren Verbinden mit einem Werkzeugelement aufweist. Ein Werkzeugelement, beispielsweise ein Sägeblatt oder ein Fräskopf, können so auf einfache Weise mit dem Maschinenelement gekoppelt werden. Ist das Werkzeugelement abgenutzt oder beschädigt, kann es auf einfache Weise wieder von dem Maschinenelement getrennt und durch ein funktionsfähiges Werkzeugelement ersetzt werden.

Günstigerweise ist die medizinische Vorrichtung in Form einer Säge, einer Fräse oder einer Bohrmaschine ausgebildet. Derartige Maschinen lassen sich insbesondere für chirurgische Zwecke einsetzen.

Die nachfolgende Beschreibung bevorzugter Ausführungsformen der Erfindung dient im Zusammenhang mit den Zeichnungen der näheren Erläuterung. Es zeigen:
- Figur 1:: eine schematische, teilweise durchbrochene perspektivische Gesamtansicht einer medizinischen Vorrichtung;
- Figur 2:: eine teilweise durchbrochene Explosionsdarstellung eines Teils der Vorrichtung aus Figur 1;
- Figur 3:: eine teilweise Schnittansicht der Vorrichtung aus Figur 1 im Bereich einer Linearführung;
- Figur 4:: eine vergrößerte Teilansicht der Anordnung aus Figur 3;
- Figur 5:: ein weiteres Beispiel eines Lagerkäfigs;
- Figur 6:: ein weiteres Beispiel eines Lagerkäfigs;
- Figur 7:: ein weiteres Beispiel eines Lagerkäfigs; und
- Figur 8:: ein erfindungsgemäßes Ausführungsbeispiel eines Lagerkäfigs.

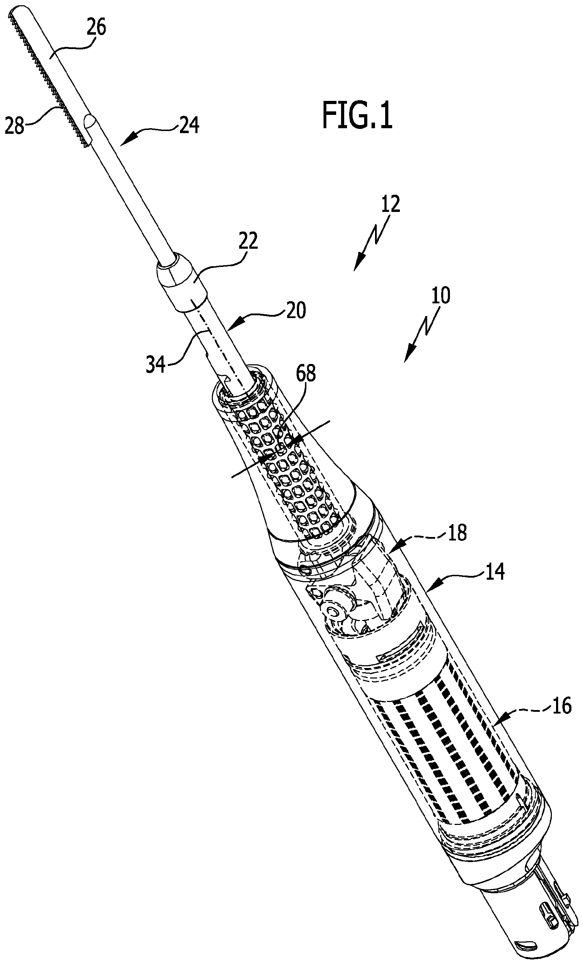

In Figur 1 ist ein Beispiel einer insgesamt mit dem Bezugszeichen 10 bezeichneten medizinischen Vorrichtung in Form einer Stichsäge 12 schematisch dargestellt.

Die Vorrichtung 10 umfasst ein als Handgriff dienendes Gehäuse 14, in welchem ein Antrieb 16 in Form eines Elektromotors angeordnet ist. Dieser kann wahlweise netzunabhängig mittels einer nicht dargestellten Batterie, insbesondere einer wiederaufladbaren Batterie, oder netzabhängig mittels eines mit dem Antrieb 16 elektrisch leitend verbundenen Netzanschlusskabel mit elektrischer Energie versorgt werden.

Statt eines Elektromotors kann auch ein Druckluftmotor oder ein anderer sterilisierbarer Antrieb zum Einsatz kommen.

Optional kann mit dem Antrieb 16 ein Getriebe 18 gekoppelt sein, um beispielsweise eine Rotation einer nicht dargestellten Antriebswelle des Antriebs 16 in eine lineare Oszillationsbewegung umzusetzen.

Mit dem Getriebe 18 kann insbesondere ein Maschinenelement 20 gekoppelt sein, welches an seinem vom Getriebe 18 weg weisenden Ende eine Kupplungseinrichtung 22 zum lösbaren Verbinden des Maschinenelements 20 mit einem Werkzeugelement 24 aufweist.

Optional kann das Maschinenelement 20 auch direkt mit dem Antrieb 16 gekoppelt sein. Auch könnte eine Kopplung des Maschinenelements 20 mit einer rotierenden biegbaren Welle vorgesehen sein.

Bei der beispielhaft in Figur 1 dargestellten medizinischen Vorrichtung 10 ist das Werkzeugelement 24 in Form eines Sägeblatts 26 mit einer eine Mehrzahl von Zähnen umfassenden Zahnreihe 28 ausgebildet. Die Kupplungseinrichtung 22 kann insbesondere in Form einer Schnellkupplung ausgebildet sein, um einen schnellen Wechsel des Sägeblatts 26 zu ermöglichen, beispielsweise wenn dieses abgenutzt ist oder durch ein anderes Sägeblatt mit anders geformten Zähnen ersetzt werden soll.

Das Maschinenelement 20 ist in Form eines Stößels 30 ausgebildet und weist einen rotationssymmetrischen Maschinenelementabschnitt 32 auf. Ein Querschnitt desselben ist kreisförmig. Der Maschinenelementabschnitt 32 definiert eine Längsachse 34.

Das Maschinenelement 20 wird durch den Antrieb 16 in Verbindung mit dem Getriebe 18 oszillierend und parallel zur Längsachse 34 hin und her bewegt.

Um das aus einem vorderen Teil des Gehäuses 14 teilweise herausragende Maschinenelement 20 in definierter Weise so zu führen, dass es mit einer koaxial zur Längsachse 34 ausgebildeten Durchbrechung 36, die es durchsetzt, nicht in Kontakt treten kann, ist eine Linearführung 38 im Gehäuse 14 angeordnet, und zwar koaxial zur Längsachse 34.

Die Linearführung 38 umfasst ein Kugellager 40 mit einer Mehrzahl von Kugeln 42, die in Kugelaufnahmen 44 beweglich gehalten oder gelagert sind, so dass sie insbesondere um ihre Kugelmittelpunkte rotieren können.

Das Kugellager 40 umfasst ferner einen Kugel- oder Lagerkäfig 46, welcher die Kugelaufnahmen 44 umfasst. Die Kugelaufnahmen 44 sind in Form von Langlöchern 48 ausgebildet. Diese werden nachfolgend noch näher beschrieben.

Die Kugeln 42 weisen einen Durchmesser 50 auf, welcher etwa doppelt so groß ist wie eine Dicke 52 einer Wand 54 des in Form einer langestreckten Hülse 56 ausgebildeten Lagerkäfigs 46.

Das Kugellager 40 umfasst ferner eine konzentrisch zur Längsachse 34 angeordnete und den Lagerkäfig 46 umgebende Lagerhülse 58. Ein Innendurchmesser 60 der Lagerhülse 58 ist etwas größer als ein Außendurchmesser 62 des Lagerkäfigs 46. Der Innendurchmesser 60 und der Außendurchmesser 62 sind so aufeinander abgestimmt, dass die in den Kugelaufnahmen 44 beweglich gehaltenen Kugeln 42 an einer Innenwandfläche 64 der Lagerhülse 58 abrollen können.

Das Kugellager 40 ist in eine korrespondierend ausgebildete Kugellageraufnahme 66 im vorderen Teil des Gehäuses 14 eingesetzt.

Die Langlöcher 48 weisen eine Breite 68 in Umfangsrichtung bezogen auf die Längsachse 34 auf sowie eine Länge 70 in einer Richtung parallel zur Längsachse 34. Somit definieren die Langlöcher 48 jeweils eine Langlochlängsachse 72, die parallel zur Achse 34 verläuft.

Die Länge 70 ist größer als der Durchmesser 50 und wird vorzugsweise so gewählt, dass eine freie Bewegungslänge 74, die von den Langlöchern 48 für die Kugeln 42 vorgegeben wird, mindestens etwa einer halben Hublänge 76 des Maschinenelements 20 entspricht. Die freie Bewegungslänge 74 ergibt sich als Differenz zwischen der Länge 70 und dem Durchmesser 50.

Die Hublänge 76 entspricht derjenigen Strecke, die das Maschinenelement 20 parallel zur Längsachse 34 hin und her bewegt wird. Die beschriebene Beziehung zwischen der freien Bewegungslänge 74 und der Hublänge 76 ergibt sich, weil die Kugeln 42 einerseits an der Innenwandfläche 64 und anderseits am Maschinenelement 20 abrollen.

Vorzugsweise definieren die Langlöcher 48 jedoch eine freie Bewegungslänge 74, die mindestens 10 % größer ist als die halbe Hublänge 76. Um ganz sicher zu stellen, dass die Kugeln 42 nicht an aufeinander zu weisenden Endflächen 78 der Langlöcher 48 anschlagen können, ist es günstig, wenn die freie Bewegungslänge 74 mindestens 30 % größer gewählt wird als die halbe Hublänge 76.

Dadurch, dass sich die Kugeln 42 in den Langlöchern 48 parallel zur Langlochlängsachse 42 frei bewegen können und idealerweise nicht an den Endflächen 78 anschlagen oder an diesen in Anlage kommen, was in unerwünschter Weise zur Folge hätte, dass sie nicht am Maschinenelement 20 und der Lagerhülse 58 abrollen, sondern an diesen entlanggleiten, wird der Lagerkäfig 46 nicht parallel zur Längsachse 34 bewegt, wenn das Maschinenelement 20 hin und her oszilliert. Durch die freie Beweglichkeit der Kugeln 42 in den Langlöchern muss der Lagerkäfig 46 bei der Oszillationsbewegung des Maschinenelements 20 nicht mitbewegt werden. Dies hat den Vorteil, dass der Lagerkäfig 46 auch aus einem schweren Material hergestellt werden kann, beispielsweise einem nicht rostenden Instrumentenstahl, ohne den Verschleiß der Linearführung 38 zu erhöhen. Dadurch, dass der Lagerkäfig 46 nicht bewegt werden muss, ist weniger Energie für die Oszillationsbewegung des Maschinenelements 20 erforderlich. Es kommt also nicht mehr darauf an, die Masse des Lagerkäfigs 46 möglichst gering zu halten.

Werden die Kugelaufnahmen 44 wie im Stand der Technik bekannt in Form von Bohrungen ausgebildet, bewegen die Kugeln 42 beim Abrollen am Maschinenelement 20 den Lagerkäfig 46 mehr oder weniger synchron zu einer Oszillationsbewegung des Maschinenelements 20 mit. Dadurch kann ein Festsitzen der Kugeln 42 in den Kugelaufnahmen 44 nicht ausgeschlossen werden, was zu einer unerwünschten Gleitbewegung der Kugeln 42 insbesondere relativ zum Maschinenelement 20 und optional auch zur Lagerhülse 58 führt und nicht zu der eigentlich gewünschten Abrollbewegung. Ein unerwünschter Effekt einer solchen Gleitbewegung ist jedoch, dass sich Vertiefungen in das Maschinenelement 20 und optional auch in die Lagerhülse 58 eingraben können. Die Gefahr solcher unerwünschter Verformungen des Maschinenelements 20 und gegebenenfalls auch der Lagerhülse 58 wird durch die langlochförmige Ausbildung der Kugelaufnahmen 44 praktisch ausgeschlossen.

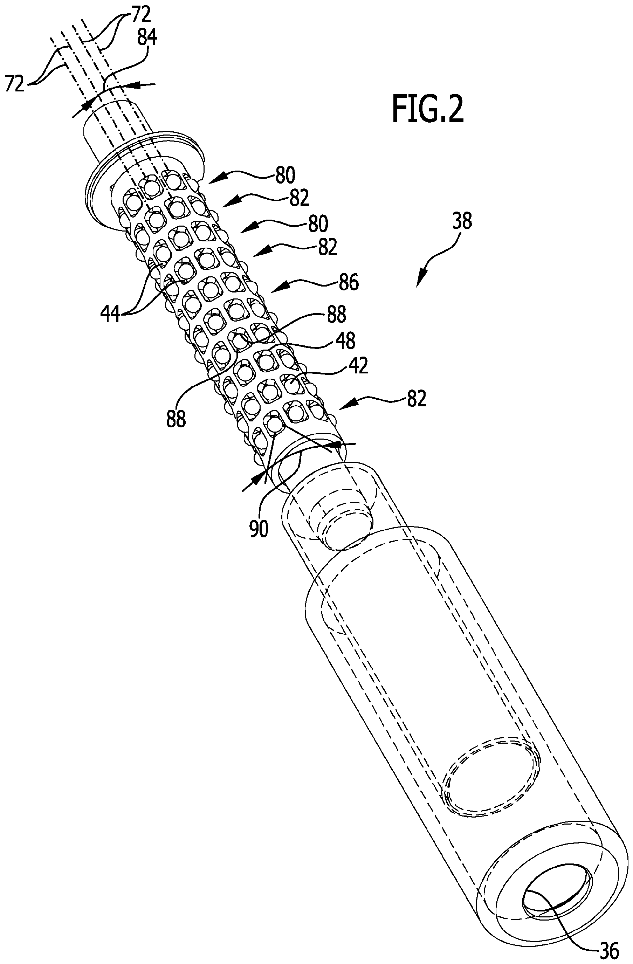

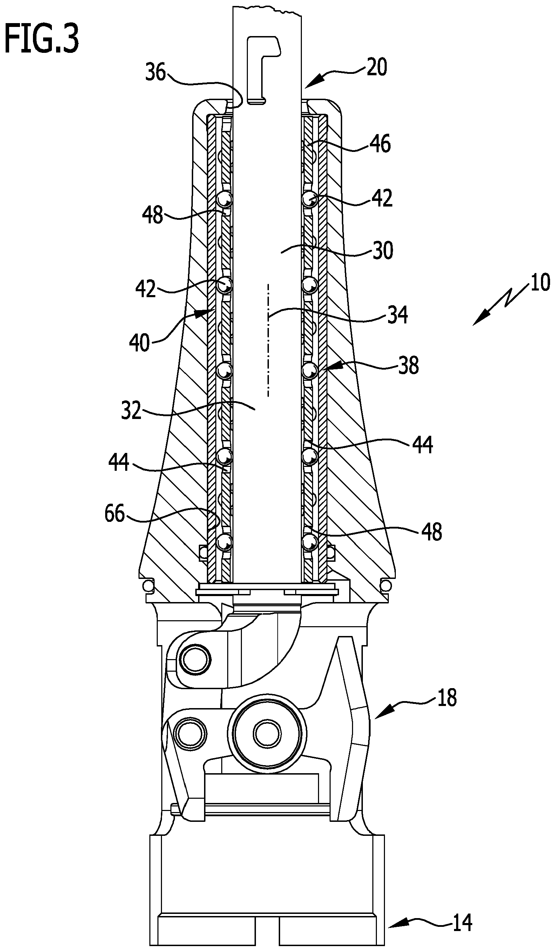

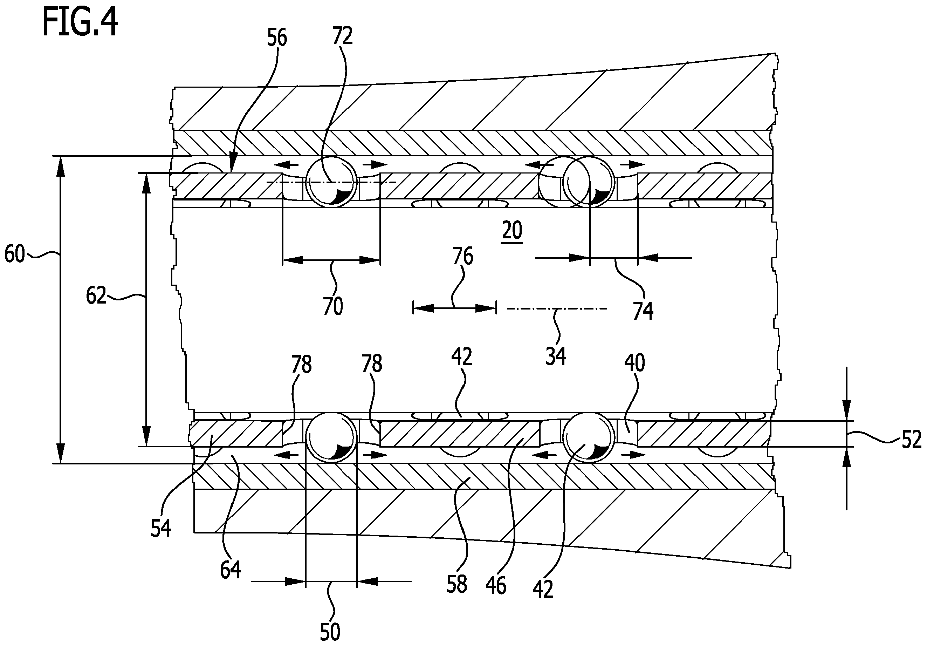

Das Kugellager 40 kann insbesondere, wie in den Figuren 1 bis 4 dargestellt, mehrere Lochreihen 80 und 82 umfassen, die jeweils mehrere Kugelaufnahmen 44 umfassen, die am Lagerkäfig 46 in Umfangsrichtung bezogen auf die Längsachse 34 ausgebildet sind.

Das in den Figuren 1 bis 4 dargestellte Beispiel J Z der Vorrichtung 10 umfasst insgesamt zehn Lochreihen, und zwar jeweils fünf Lochreihen 80 und fünf Lochreihen 82. Die Langlöcher 48 der Lochreihen 80 und 82 sind jeweils gleichmäßig über den Umfang verteilt ausgebildet. Beide Lochreihen 80 und 82 weisen jeweils dieselbe Anzahl an Kugelaufnahmen 44 auf.

Die Lochreihen 80 und 82 sind derart ausgebildet, dass Längsachsen der Langlöcher 48 der Lochreihe 80 relativ zu den Langlochlängsachsen 72 der Langlöcher 48 der Lochreihe 82 um einen halben Umfangswinkel 84, der durch einen Winkelabstand zwischen den Langlochlängsachsen 72 der Langlöcher 48 einer der beiden Lochreihen 80 oder 82 definiert wird, versetzt angeordnet sind.

Alternativ können die Langlöcher 48 der benachbarten Lochreihen 80 und 82 auch zueinander fluchtend angeordnet sein, also derart, dass die Langlochlängsachsen 72 von Langlöchern 48 benachbarter Lochreihen 80 und 82 zusammenfallen.

Die Linearführung 38 umfasst ferner eine Radialsicherungseinrichtung 86 zum Verhindern des Austretens der Kugeln 42 aus den jeweiligen Kugelaufnahmen 44 in einer Richtung auf die Längsachse 34 hin. Die Radialsicherungseinrichtung 86 umfasst die Breite 68 der Langlöcher 48 definierende seitliche Begrenzungsflächen 88, die von der Längsachse 34 weg weisend um einen Konuswinkel 90 gegen einander geneigt sind. Der Konuswinkel 90 kann insbesondere in einem Bereich zwischen 0° und etwa 45° liegen.

Durch die gegeneinander geneigten Begrenzungsflächen 88 nimmt die Breite 68 quer zur Langlochlängsachse 72 in radialer Richtung auf die Längsachse 34 hin ab. Mithin wird also die Kugelaufnahme 44 in Richtung auf die Längsachse 34 hin schmaler und weist eine minimale Langlochbreite 92 beim Durchtritt durch die Wand 54 des Lagerkäfigs 46 auf, welche minimale Langlochbreite 92 kleiner ist als der Durchmesser 50. Dadurch wird sichergestellt, dass die Kugeln 42 nicht in Richtung auf die Längsachse 34 hin aus den Kugelaufnahmen 44 herausfallen können.

Ferner bildet auch die Lagerhülse 58 einen Teil der Radialsicherungseinrichtung 86, denn sie umgibt den Lagerkäfig 46 wie bereits beschrieben derart, dass eine Bewegung der Kugeln 42 von der Längsachse 34 weg ebenfalls begrenzt wird. Die Lagerhülse 58 verhindert somit das Austreten der Kugeln 42 aus den Kugelaufnahmen 44 in einer Richtung von der Längsachse 34 weg. Zusätzlich oder auch alternativ zu relativ zueinander geneigten Begrenzungsflächen können die Begrenzungsflächen 88 und die Innenwandfläche 64 verbindende Kanten 94 verstemmt sein, also etwas in Richtung auf eine durch das Langloch 48 definierte Durchgangsöffnung des Lagerkäfigs 46 hin, so dass die minimale Langlochbreite 92 etwas verringert wird, und zwar mindestens so viel, dass diese kleiner ist als der Durchmesser 50. Auch so kann dann das Durchfallen der Kugeln 42 in Richtung auf die Längsachse 34 hin verhindert werden.

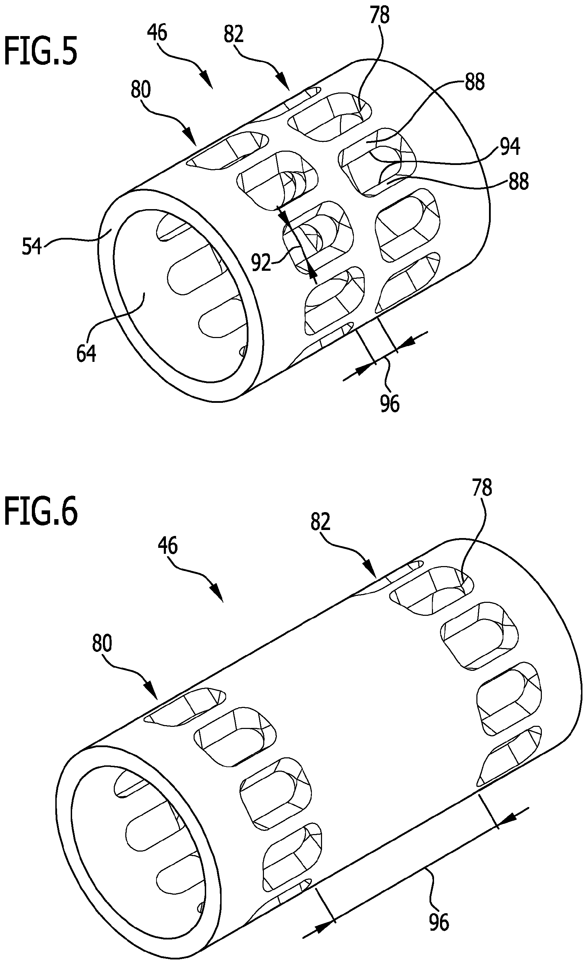

In Figur 5 ist ein weiteres Beispiel eines Lagerkäfigs 46 schematisch dargestellt. Er ist ebenfalls in Form einer langgestreckten Hülse 56 ausgebildet und umfasst lediglich zwei Lochreihen 80 und 82, deren jeweilige Langlöcher 48, ebenso wie bei dem in den Figuren 1 bis 4 dargestellten Ausführungsbeispiel des Lagerkäfigs 46, um einen halben Umfangswinkel 84 relativ zueinander versetzt ausgebildet sind.

Der Lagerkäfig 46 kann beispielsweise eingesetzt werden, um das Maschinenelement 20 lediglich in voneinander beabstandeten Abschnitten des Maschinenelementabschnitts 32 zu lagern. Auf diese Weise kann auf einen deutlich mehr als zwei Lochreihen 80 beziehungsweise 82 umfassenden Lagerkäfig 46 verzichtet werden. So lassen sich Aufwand und Kosten bei der Herstellung der Vorrichtung 10 reduzieren.

Eine besonders gute Führung lässt sich erreichen, wenn, wie bei dem in Figur 6 schematisch dargestellten Beispiel des Lagerkäfigs 46, ein Abstand 96 benachbarter Lochreihen 80 und 82 voneinander deutlich vergrößert wird. Insbesondere kann der Abstand 96 ein Mehrfaches der Länge 70 betragen. Auf diese Weise lässt sich die Herstellung des Lagerkäfigs 46 vereinfachen und die Zahl der Kugeln 42 reduzieren.

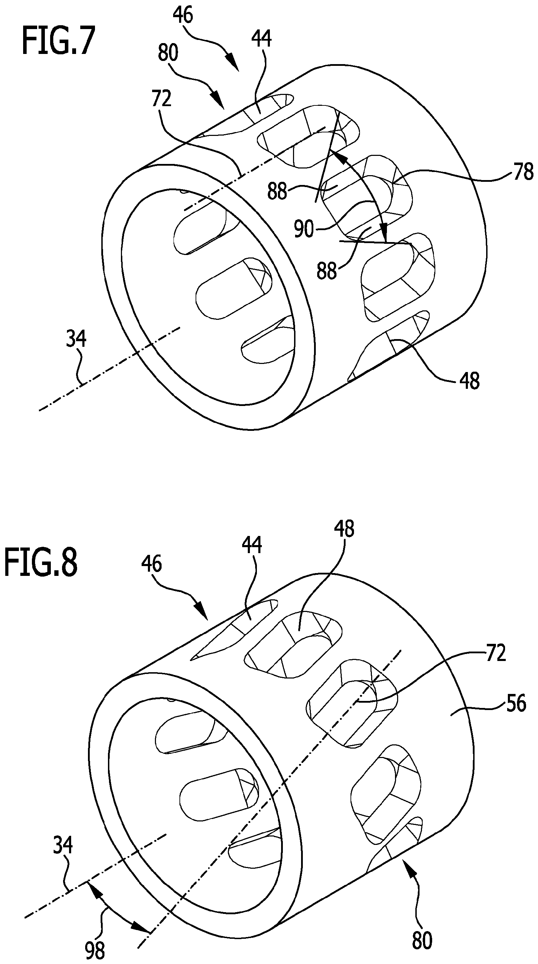

In Figur 7 ist ein weiteres Beispiel eines Lagerkäfigs 46 schematisch dargestellt. Er umfasst lediglich eine einzige, mehrere Langlöcher 48 umfassende Lochreihe 80. Die Langlöcher 48 sind mit ihren Langlochlängsachsen 72, ebenso wie bei allen vorstehend beschriebenen Ausführungsbeispielen von Kugelkäfigen 46, parallel zur Längsachse 34 des Lagerkäfigs 46 ausgerichtet. Für die Lagerung des in den Figuren 1 bis 4 dargestellten Maschinenelements 20 können beispielsweise zwei der in Figur 7 dargestellten Lagerkäfige 46 zum Einsatz kommen, die dann im Wesentlichen so positioniert werden, dass deren Lochreihen 80 das Maschinenelement 20 etwa an den Positionen umgeben, wo die bei dem in den Figuren 1 bis 4 dargestellten Lagerkäfig 46 am weitesten voneinander entfernten Lochreihen 80 und 82 positioniert sind. So lassen sich signifikant Kosten bei der Herstellung des Lagerkäfigs 46 und damit auch für die Linearführung 38 einsparen.

In Figur 8 ist ein erfindungsgemäßes Ausführungsbeispiel eines Lagerkäfigs 46 schematisch dargestellt. Es umfasst wiederum eine Hülse 56, die eine mehrere Langlöcher 48 umfassende Lochreihe 80 umfasst. Die Langlöcher 48 sind im Wesentlichen identisch ausgebildet wie die Langlöcher bei dem in Figur 7 dargestellten Ausführungsbeispiel des Lagerkäfigs 46. Allerdings sind die Langlöcher 48 mit ihren Langlochlängsachsen 72 relativ zur Längsachse 34 um einen Drallwinkel 98 geneigt. Dieser liegt vorzugsweise in einem Bereich zwischen 0° und etwa 30°. Die so relativ zur Längsachse 34 quer oder schräg verlaufenden Langlöcher 48 haben den positiven Effekt, dass in Folge einer Hin- und Herbewegung des Maschinenelements 20 parallel zur Längsachse 34 der Lagerkäfig 46 durch die Bewegung der Kugeln 42 in Umfangsrichtung hin und her bewegt wird. So kann es insgesamt zu einer Drehbewegung des Lagerkäfigs 46 um die Längsachse 34 oder zu einer oszillierenden Drehbewegung des Lagerkäfigs 46 um die Längsachse 34 kommen. Dadurch kann mit relativ hoher Zuverlässigkeit vermieden werden, dass sich Reib- oder Schleifspuren am Maschinenelement 20 und optional auch an der Lagerhülse 58 parallel zur Längsachse 34 ausbilden können. Wenn es doch zu Reibbewegungen der Kugeln 42 relativ zum Maschinenelement 20 und optional auch zur Lagerhülse 58 kommen sollte, verlaufen diese jedoch nicht immer an identischen Positionen, sondern über den Umfang des Maschinenelements 20 und gegebenenfalls auch der Lagerhülse 58 verteilt.

Mit den Langlochlängsachsen 72 relativ zur Längsachse 34 schräg verlaufenden Langlöchern 48 können auch bei Lagerkäfigen 46 mit zwei oder noch mehr Lochreihen 80 beziehungsweise 82 vorgesehen werden, insbesondere auch bei den oben beschriebenen anderen Beispiel der Figuren 1 bis 7, und zwar entweder ausschließlich oder aber auch nur teilweise.

Die oben beschriebene erfindungsgemäße Ausführungsform und die obigen Beispiele von Lagerkäfigen 46 und Formen von Langlöchern 48 lassen sich im Wesentlichen beliebig miteinander kombinieren, so dass zur Führung eines Maschinenelements 20 stets eine optimal angepasste Linearführung 38 bereitgestellt werden kann. So können insbesondere die Anzahl der Kugeln 42 sowie die Anzahl der zur Führung des Maschinenelements 20 eingesetzten Kugellager 40 von der Länge des Maschinenelements 20 beziehungsweise des Maschinenelementabschnitts 32 parallel zur Längsachse 34 abhängen als auch von einer Oszillationsfrequenz des Maschinenelements 20.

Allen oben beschriebenen Lagerkäfigen 46 ist insbesondere gemein, dass eine Gleitbewegung der in ihnen gelagerten Kugeln 42 keine Bewegung der Lagerkäfige 46 parallel zur Längsachse 34 bewirkt. Dadurch können sie, wie bereits beschrieben, auch aus einem Material mit einer hohen Massendichte hergestellt werden, beispielsweise aus einem nichtrostenden Instrumentenstahl. Selbstverständlich können die Lagerkäfige auch aus Kunststoffen oder Keramiken ausgebildet werden.

### Bezugszeichenliste

- 10: Medizinische Vorrichtung
- 12: Stichsäge
- 14: Gehäuse
- 16: Antrieb
- 18: Getriebe
- 20: Maschinenelement
- 22: Kupplungseinrichtung
- 24: Werkzeugelement
- 26: Sägeblatt
- 28: Zahnreihe
- 30: Stößel
- 32: Maschinenelementabschnitt
- 34: Längsachse
- 36: Durchbrechung
- 38: Linearführung
- 40: Kugellager
- 42: Kugel
- 44: Kugelaufnahme
- 46: Lagerkäfig
- 48: Langloch
- 50: Durchmesser
- 52: Dicke
- 54: Wand
- 56: Hülse
- 58: Lagerhülse
- 60: Innendurchmesser
- 62: Außendurchmesser
- 64: Innenwandfläche
- 66: Kugellageraufnahme
- 68: Breite
- 70: Länge
- 72: Langlochlängsachse
- 74: freie Bewegungslänge
- 76: Hublänge
- 78: Endfläche
- 80: Lochreihe
- 82: Lochreihe
- 84: Umfangswinkel
- 86: Radialsicherungseinrichtung
- 88: Begrenzungsfläche
- 90: Konuswinkel
- 92: Langlochbreite
- 94: Kante
- 96: Abstand
- 98: Drallwinkel

## Patentansprüche

1. Medizinische Vorrichtung (10) mit einem linear beweglich gelagerten Maschinenelement (20) und mindestens einer mindestens ein Kugellager (40) umfassenden, eine Längsachse (34) definierenden Linearführung (38) zum Führen einer linearen oszillierenden Hin- und Herbewegung des Maschinenelements (20) parallel zur Längsachse (34) mit einer hohen Oszillationsfrequenz, welches mindestens eine Kugellager (40) eine Mehrzahl von Kugeln (42) umfasst, die in korrespondierenden Kugelaufnahmen (44) beweglich gehalten sind und die das Maschinenelement (20) führen, wobei die Mehrzahl von Kugelaufnahmen (44) in Form von Langlöchern (48) ausgebildet ist, wobei die Mehrzahl von Langlöchern (48) eine freie Bewegungslänge (74) für die jeweils in ihnen beweglich angeordnete Kugel (42) aufweist und wobei die freie Bewegungslänge (74) mindestens einem halben Hub (76) des Maschinenelements (20) relativ zur Linearführung (38) entspricht, wobei die Langlöcher (48) jeweils eine Langlochlängsachse (72) definieren, **dadurch gekennzeichnet, dass** die Langlochlängsachse (72) quer zur Längsachse (34) verläuft, dass die Langlochlängsachsen (72) mit der Längsachse (34) einen Winkel (98) in einem Bereich zwischen 0° und 90° einschließen und dass die medizinische, insbesondere sterilisierbare, Vorrichtung (10) ausgebildet ist, um mit einer Reinigungsflüssigkeit gereinigt zu werden.

2. Medizinische Vorrichtung nach Anspruch 1, **dadurch gekennzeichnet, dass** das Maschinenelement (20) mindestens einen rotationssymmetrischen Maschinenelementabschnitt (32) umfasst, welcher in der mindestens einen Linearführung (38) geführt ist,
wobei insbesondere der rotationssymmetrische Maschinenelementabschnitt (32) einen kreisförmigen Querschnitt aufweist.

3. Medizinische Vorrichtung nach einem der voranstehenden Ansprüche, **dadurch gekennzeichnet, dass** das Maschinenelement (20) in Form eines Stößels (30) oder eines Kolbens ausgebildet ist.

4. Medizinische Vorrichtung nach einem der voranstehenden Ansprüche, **dadurch gekennzeichnet, dass** das mindestens eine Kugellager (40) einen Lagerkäfig (46) umfasst und dass die Mehrzahl von Kugelaufnahmen (44) im Lagerkäfig (46) ausgebildet ist.

5. Medizinische Vorrichtung nach einem der voranstehenden Ansprüche, **dadurch gekennzeichnet, dass** die Langlochlängsachsen (72) mit der Längsachse (34) einen Drallwinkel (98) in einem Bereich zwischen 0° bis etwa 30° einschließen.

6. Medizinische Vorrichtung nach einem der voranstehenden Ansprüche, **dadurch gekennzeichnet, dass** die Langlöcher (48) eine freie Bewegungslänge (74) aufweisen, die mindestens 10 % größer ist als der halbe Hub (76), insbesondere mindestens 30 % größer ist als der halbe Hub (76).

7. Medizinische Vorrichtung nach einem der Ansprüche 4 bis 6, **dadurch gekennzeichnet, dass**
a) der Lagerkäfig (46) rotierbar um die Längsachse (34) an der Linearführung (38) angeordnet ist
und/oder
b) das mindestens eine Kugellager (40) eine den Lagerkäfig (46) umgebende Lagerhülse (58) aufweist zum Begrenzen einer Bewegung der Kugeln (42) in radialer Richtung von der Längsachse (34) weg.

8. Medizinische Vorrichtung nach einem der voranstehenden Ansprüche, **dadurch gekennzeichnet, dass** die Mehrzahl von Langlöchern (48) eine Langlochbreite (68) quer zu ihrer jeweiligen Langlochlängsachse (72) aufweist und dass die Langlochbreite (68) in radialer Richtung auf die Längsachse (34) hin abnimmt.

9. Medizinische Vorrichtung nach einem der voranstehenden Ansprüche, **dadurch gekennzeichnet, dass** aufeinander zu weisende seitliche Begrenzungsflächen (88) der Mehrzahl von Langlöchern (48) um einen Konuswinkel (90) gegeneinander geneigt sind,
wobei insbesondere der Konuswinkel (90) von der Längsachse (34) weg weisend geöffnet ist.

10. Medizinische Vorrichtung nach einem der Ansprüche 4 bis 9, **dadurch gekennzeichnet, dass**
a) eine Wandstärke (52) des Lagerkäfigs (46) kleiner als ein Durchmesser (50) der Mehrzahl von Kugeln (42) ist, insbesondere entspricht die Wandstärke (52) maximal einem halben Durchmesser (50) der Mehrzahl von Kugeln,
und/oder
b) der Lagerkäfig (46) mindestens zwei in Umfangsrichtung bezogen auf dessen Längsachse (34) axial voneinander beabstandete, jeweils mehrere Langlöcher (48) umfassende Lochreihen (80, 82) aufweist,
wobei insbesondere Langlöcher (48) benachbarter Lochreihen (80, 82) zueinander fluchtend oder versetzt angeordnet sind.

11. Medizinische Vorrichtung nach einem der Ansprüche 4 bis 10, **dadurch gekennzeichnet, dass** die Linearführung (38) zwei oder mehr voneinander getrennte Kugellager (40) mit jeweils mindestens einem Lagerkäfig (46) umfasst und dass jeder Lagerkäfig (46) mindestens eine Lochreihe (80, 82) aufweist mit mehreren in Umfangsrichtung bezogen auf dessen Längsachse (34) angeordneten Langlöchern (48).

12. Medizinische Vorrichtung nach einem der Ansprüche 4 bis 11, **dadurch gekennzeichnet, dass** der Lagerkäfig (46) eine Radialsicherungseinrichtung (86) aufweist zum Verhindern des Austretens der Kugeln (42) aus den jeweiligen Kugelaufnahmen (44) in einer Richtung von der Längsachse (34) weg und/oder in einer Richtung auf die Längsachse (34) hin.

13. Medizinische Vorrichtung nach Anspruch 12, **dadurch gekennzeichnet, dass** die Radialsicherungseinrichtung (86)
a) verstemmte Ränder (94) der Kugelaufnahmen (44) umfasst und/oder
b) eine Deckelhülse umfasst, deren Innendurchmesser zu einem Außendurchmesser des Lagerkäfigs (46) korrespondiert, und dass die Deckelhülse eine Mehrzahl von langlochartigen Deckeldurchbrechungen aufweist, deren Breite kleiner ist als ein Kugeldurchmesser (50),
wobei insbesondere die Lagerhülse (58) die Deckelhülse umgebend angeordnet ist.

14. Medizinische Vorrichtung nach einem der voranstehenden Ansprüche, **dadurch gekennzeichnet, dass**
a) das mindestens eine Kugellager (40), insbesondere der Lagerkäfig (46), aus einem Metall ausgebildet ist, insbesondere aus Stahl, und/oder
b) das Maschinenelement (20) eine Kupplungseinrichtung (22) zum lösbaren Verbinden mit einem Werkzeugelement (24) aufweist.

15. Medizinische Vorrichtung nach einem der voranstehenden Ansprüche, **dadurch gekennzeichnet, dass** sie in Form einer Säge (12), einer Fräse oder einer Bohrmaschine ausgebildet ist,
wobei insbesondere die medizinische Vorrichtung einen sterilisierbaren Antrieb umfasst,
wobei weiter insbesondere der sterilisierbare Antrieb ein Elektromotor oder ein Druckluftmotor ist.

## Claims

1. Medical device (10) with a machine element (20) mounted so as to be linearly movable and at least one linear guide (38), comprising at least one ball bearing (40) and defining a longitudinal axis (34), for guiding a linear oscillating movement back and forth of the machine element (20) parallel to the longitudinal axis (34) at a high high oscillation frequency, said at least one ball bearing (40) comprising a plurality of balls (42) which are movably held in corresponding ball holders (44) and which guide the machine element (20), wherein the plurality of ball holders (44) are constructed in the form of elongated holes (48), wherein the plurality of elongated holes (48) have a free length of movement (74) for the ball (42) movably arranged in each of them, and wherein the free length of movement (74) corresponds to at least half of a stroke (76) of the machine element (20) relative to the linear guide (38), wherein the elongated holes (48) each define an elongated hole longitudinal axis (72), **characterized in that** the elongated hole longitudinal axis (72) extends transversely to the longitudinal axis (34), **in that** the elongated hole longitudinal axes (72) include with the longitudinal axis (34) an angle (98) in a range between 0° and 90°, and **in that** the medical, in particular sterilizable, device (10) is constructed so as to be cleanable with a cleaning liquid.

2. Medical device in accordance with claim 1, **characterized in that** the machine element (20) comprises at least one rotationally symmetrical machine element section (32) which is guided in the at least one linear guide (38),
wherein, in particular, the rotationally symmetrical machine element section (32) has a circular cross section.

3. Medical device in accordance with any one of the preceding claims, **characterized in that** the machine element (20) is constructed in the form of a ram (30) or a piston.

4. Medical device in accordance with any one of the preceding claims, **characterized in that** the at least one ball bearing (40) comprises a bearing cage (46), and **in that** the plurality of ball holders (44) are formed in the bearing cage (46).

5. Medical device in accordance with any one of the preceding claims, **characterized in that** the elongated hole longitudinal axes (72) include with the longitudinal axis (34) an angle of twist (98) in a range between 0° to approximately 30°.

6. Medical device in accordance with any one of the preceding claims, **characterized in that** the elongated holes (48) have a free length of movement (74) which is at least 10 % greater than half of the stroke (76), in particular, at least 30 % greater than half of the stroke (76).

7. Medical device in accordance with any one of claims 4 to 6, **characterized in that**
a) the bearing cage (46) is arranged on the linear guide (38) for rotation about the longitudinal axis (34)
and/or
b) the at least one ball bearing (40) comprises a bearing sleeve (58) surrounding the bearing cage (46) for limiting movement of the balls (42) in the radial direction away from the longitudinal axis (34).

8. Medical device in accordance with any one of the preceding claims, **characterized in that** the plurality of elongated holes (48) have an elongated hole width (68) transversely to their respective elongated hole longitudinal axis (72), and **in that** the elongated hole width (68) decreases in the radial direction towards the longitudinal axis (34)

9. Medical device in accordance with any one of the preceding claims, **characterized in that** lateral delimitation surfaces (88) of the plurality of elongated holes (48) that face each other are inclined at a cone angle (90) to each other,
wherein, in particular, the cone angle (90) is open facing away from the longitudinal axis (34).

10. Medical device in accordance with any one of claims 4 to 9, **characterized in that**
a) a wall thickness (52) of the bearing cage (46) is smaller than a diameter (50) of the plurality of balls (42), in particular, the wall thickness (52) corresponds at most to half of a diameter (50) of the plurality of balls
and/or
b) the bearing cage (46) comprises at least two rows of holes (80, 82) axially spaced from each other in the circumferential direction in relation to its longitudinal axis (34) and each comprising several elongated holes (48),
wherein, in particular, elongated holes (48) of adjacent rows of holes (80, 82) are arranged in alignment with or offset from one another.

11. Medical device in accordance with any one of claims 4 to 10, **characterized in that** the linear guide (38) comprises two or more ball bearings (40) separate from each other with at least one bearing cage (46) each, and **in that** each bearing cage (46) has at least one row of holes (80, 82) with several elongated holes (48) arranged in the circumferential direction in relation to its longitudinal axis (34).

12. Medical device in accordance with any one of claims 4 to 11, **characterized in that** the bearing cage (46) comprises a radial securing device (86) for preventing the balls (42) from exiting from the respective ball holders (44) in a direction away from the longitudinal axis (34) and/or in a direction towards the longitudinal axis (34).

13. Medical device in accordance with claim 12, **characterized in that** the radial securing device (86)
a) comprises bent edges (94) of the ball holders (44)
and/or
b) comprises a cover sleeve, the inner diameter of which corresponds to an outer diameter of the bearing cage (46), and **in that** the cover sleeve comprises a plurality of elongated hole-like cover through-holes, the width of which is smaller than a ball diameter (50),
wherein, in particular, the bearing sleeve (58) is arranged so as to surround the cover sleeve.

14. Medical device in accordance with any one of the preceding claims, **characterized in that**
a) the at least one ball bearing (40), in particular, the bearing cage (46), is formed from a metal, in particular, from steel,
and/or
b) the machine element (20) comprises a coupling device (22) for releasable connection to a tool element (24).

15. Medical device in accordance with any one of the preceding claims, **characterized in that** it is constructed in the form of a saw (12), a milling machine or a drilling machine,
wherein, in particular, the medical device comprises a sterilizable drive, wherein, further in particular, the sterilizable drive is an electric motor or a compressed-air motor.

## Revendications

1. Dispositif médical (10) avec un élément de machine (20) monté mobile linéairement et au moins un guide linéaire (38) comprenant au moins un roulement à billes (40) et définissant un axe longitudinal (34) pour guider un mouvement de va-et-vient oscillant linéaire de l'élément de machine (20) parallèle à l'axe longitudinal (34) avec une fréquence d'oscillation élevée, lequel au moins un roulement à billes (40) comprend une pluralité de billes (42) qui sont maintenues de manière mobile dans des logements de billes (44) correspondants et qui guident l'élément de machine (20), où la pluralité de logements de billes (44) est réalisée sous la forme de trous allongés (48), où la pluralité de trous allongés (48) présente une longueur de mouvement libre (74) pour les billes (42) disposées chacune de manière mobile dans ceux-ci et où la longueur de mouvement libre (74) correspond à au moins une demi-course (76) de l'élément de machine (20) par rapport au guide linéaire (38), où les trous allongés (48) définissent chacun un axe longitudinal de trou allongé (72), **caractérisé en ce que** l'axe longitudinal de trou allongé (72) s'étend transversalement à l'axe longitudinal (34), **en ce que** les axes longitudinaux de trous allongés (72) forment avec l'axe longitudinal (34) un angle (98) dans une plage entre 0° et 90° et **en ce que** le dispositif médical (10), en particulier stérilisable, est réalisé pour être nettoyé avec un liquide de nettoyage.

2. Dispositif médical selon la revendication 1, **caractérisé en ce que** l'élément de machine (20) comprend au moins un tronçon d'élément de machine à symétrie de rotation (32) qui est guidé dans le au moins un guide linéaire (38),
où en particulier le tronçon d'élément de machine à symétrie de rotation (32) présente une section transversale circulaire.

3. Dispositif médical selon l'une des revendications précédentes, **caractérisé en ce que** l'élément de machine (20) est réalisé sous la forme d'un poussoir (30) ou d'un piston.

4. Dispositif médical selon l'une des revendications précédentes, **caractérisé en ce que** le au moins un roulement à billes (40) comprend une cage de roulement (46) et **en ce que** la pluralité de logements de billes (44) est réalisée dans la cage de roulement (46).

5. Dispositif médical selon l'une des revendications précédentes, **caractérisé en ce que** les axes longitudinaux de trous allongés (72) forment avec l'axe longitudinal (34) un angle de torsion (98) dans une plage entre 0° et 30° environ.

6. Dispositif médical selon l'une des revendications précédentes, **caractérisé en ce que** les trous allongés (48) présentent une longueur de mouvement libre (74) qui est supérieure d'au moins 10 % à la demi-course (76), en particulier supérieure d'au moins 30 % à la demi-course (76).

7. Dispositif médical selon l'une des revendications 4 à 6, **caractérisé en ce que**
a) la cage de roulement (46) est disposée sur le guide linéaire (38) de manière à pouvoir tourner autour de l'axe longitudinal (34) et/ou
b) le au moins un roulement à billes (40) présente une gaine de roulement (58) entourant la cage de roulement (46) pour limiter un mouvement des billes (42) dans la direction radiale en s'éloignant de l'axe longitudinal (34).

8. Dispositif médical selon l'une des revendications précédentes, **caractérisé en ce que** la pluralité de trous allongés (48) présente une largeur de trou allongé (68) transversalement à leur axe longitudinal de trou allongé (72) respectif et **en ce que** la largeur de trou allongé (68) diminue dans la direction radiale vers l'axe longitudinal (34).

9. Dispositif médical selon l'une des revendications précédentes, **caractérisé en ce que** des surfaces latérales de limitation (88) de la pluralité de trous allongés (48) tournées les unes vers les autres sont inclinées les unes par rapport aux autres d'un angle de cône (90),
où en particulier l'angle de cône (90) est ouvert à l'opposé de l'axe longitudinal (34).

10. Dispositif médical selon l'une des revendications 4 à 9, **caractérisé en ce que**
a) une épaisseur de paroi (52) de la cage de roulement (46) est inférieure à un diamètre (50) de la pluralité de billes (42),
en particulier l'épaisseur de paroi (52) correspond au maximum à un demi-diamètre (50) de la pluralité de billes,
et/ou
b) la cage de roulement (46) présente au moins deux rangées de trous (80, 82) espacées axialement l'une de l'autre dans la direction circonférentielle par rapport à son axe longitudinal (34) comprenant chacune plusieurs trous allongés (48),
où en particulier les trous allongés (48) de rangées de trous (80, 82) adjacentes sont disposés alignés ou décalés les uns par rapport aux autres.

11. Dispositif médical selon l'une des revendications 4 à 10, **caractérisé en ce que** le guide linéaire (38) comprend deux ou plusieurs roulements à billes (40) séparés les uns des autres avec chacun au moins une cage de roulement (46) et **en ce que** chaque cage de roulement (46) présente au moins une rangée de trous (80, 82) avec plusieurs trous allongés (48) disposés dans la direction circonférentielle par rapport à son axe longitudinal (34).

12. Dispositif médical selon l'une des revendications 4 à 11, **caractérisé en ce que** la cage de roulement (46) présente un dispositif d'arrêt radial (86) pour empêcher la sortie des billes (42) des logements de billes (44) respectifs dans une direction s'éloignant de l'axe longitudinal (34) et/ou dans une direction vers l'axe longitudinal (34).

13. Dispositif médical selon la revendication 12, **caractérisé en ce que** le dispositif d'arrêt radial (86)
a) comprend des bords rabattus (94) des logements de billes (44)
et/ou
b) comprend une gaine de recouvrement dont le diamètre interne correspond à un diamètre externe de la cage de roulement (46), et **en ce que** la gaine de recouvrement présente une pluralité d'ouvertures de recouvrement en forme de trou allongé, dont la largeur est inférieure à un diamètre de bille (50),
où en particulier la gaine de roulement (58) est disposée autour de la gaine de recouvrement.

14. Dispositif médical selon l'une des revendications précédentes, **caractérisé en ce que**
a) le au moins un roulement à billes (40), en particulier la cage de roulement (46), est formé d'un métal, en particulier d'acier,
et/ou
b) l'élément de machine (20) présente un dispositif d'accouplement (22) pour la liaison amovible avec un élément d'outil (24).

15. Dispositif médical selon l'une des revendications précédentes, **caractérisé en ce qu'**il est réalisé sous la forme d'une scie (12), d'une fraise ou d'une perceuse,
où en particulier le dispositif médical comprend un entraînement stérilisable,
où en particulier également l'entraînement stérilisable est un moteur électrique ou un moteur à air comprimé.
